(19) Europäisches Patentamt
European Patent Office
Office européen des brevets

(11) Veröffentlichungsnummer : **0 125 542**
**B1**

(12) # EUROPÄISCHE PATENTSCHRIFT

(45) Veröffentlichungstag der Patentschrift : **02.03.88**

(51) Int. Cl.[4] : **C 07 C 29/00**, C 07 C143/68

(21) Anmeldenummer : **84104707.9**

(22) Anmeldetag : **26.04.84**

(54) **Verfahren zur Herstellung von Hydrochloriden.**

(30) Priorität : **02.05.83 HU 149783**

(43) Veröffentlichungstag der Anmeldung : **21.11.84 Patentblatt 84/47**

(45) Bekanntmachung des Hinweises auf die Patenterteilung : **02.03.88 Patentblatt 88/09**

(84) Benannte Vertragsstaaten : **AT BE CH DE FR GB IT LI NL SE**

(56) Entgegenhaltungen :
**DD-A- 32 304**
**GB-A- 1 374 390**
**US-A- 3 745 188**

(73) Patentinhaber : **BIOGAL GYOGYSZERGYAR**
**Pallagi u. 13.**
**H-4042 Debrecen (HU)**

(72) Erfinder : **Szarvas, Niklòs, Dr. Dipl. Chem.**
**Peterfia u. 40**
**H-4042 Debrecen (HU)**
Erfinder : **Horváth née Fehér, Eva, Dr. Dipl. Chem.**
**Naphegy u. 33.II.5**
**H-1016 Budapest (HU)**
Erfinder : **Cséke, Lászlò, Dipl. Chem.**
**Hajò u. 4**
**H-4042 Debrecen (HU)**
Erfinder : **Bálint, János, Dr. Dipl. Chem.**
**Péchy u. 5**
**H-4042 Debrecen (HU)**
Erfinder : **Fábián, Ferenc**
**Hollò J. u. 4**
**H-4042 Debrecen (HU)**
Erfinder : **Kun, Lajos**
**Szegfü u. 6**
**H-4042 Debrecen (HU)**

(74) Vertreter : **Hansen, Bernd, Dr.rer.nat. et al**
**Hoffmann, Eitle & Partner Patentanwälte Arabellastrasse 4**
**D-8000 München 81 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 125 542 B1

Jouve, 18, rue St-Denis, 75001 Paris, France

**Beschreibung**

Die Erfindung betrifft ein neues Verfahren zur Herstellung von Hydrochloriden, d.h. den mit Salzsäure gebildeten Säureadditionssalzen von Verbindungen, die ein protonierbares Stickstoffatom enthalten.

Die Hydrochloride von Verbindungen, die ein protonierbares Stickstoffatom enthalten, sind polare, gut wasserlösliche Substanzen, die auf vielan Gebieten des praktischen Lebens, zum Beispiel in der Heilkunde, bei der Herstellung von Kunststoffen, im Pflanzenschutz, in Farbstoffen, als analytische Reagentien usw., weitverbreitet angewendet werden. Für die Verbreitung der Hydrochloride ist charakteristisch, daß etwa 10 % aller im Merck-Index (Ninth Ed., Ed. by M. Windholz, Merck, Rahway, USA 1976) aufgeführten Arzneimittelwirkstoffe, d. h. etwa 900 Verbindungen, in Form ihrer Hydrochloride als Heilmittelwirkstoffe verwendet werden.

Die Hydrochloride dieser Verbindungen werden im allgemeinen in Wasser oder einem wäßrigen organischen Lösungsmittel, meistens einem Alkohol, durch Zugabe von Salzsäure hergestellt. Dies ist in den britischen Patentschriften Nr. 718.020 und 718.032 für das Hydrochlorid des Oxytetracyclins beschrieben. Eine weitere Möglichkeit zur Bildung des Hydrochlorids besteht darin, die Ausgangsverbindung in einem organischen Lösungsmittel, zum Beispiel einem Alkohol, zu lösen oder zu suspendieren und in die Lösung beziehungsweise Suspension Salzsäuregas einzuleiten. Man kann die Salzbildung auch in einem organischen Lösungsmittel vornehmen, in dem vorher Salzsäuregas gelöst wurde. Auf diese Weise wird zum Beispiel gemäß der belgischen Patentschrift 638.881 und der ungarischen Patentschrift Nr. 143.911 das erwähnte Oxytetracyclin-hydrochlorid hergestellt ; dabei werden die Löslichkeitsverhaltnisse der Ausgangs- und/oder der Zielverbindung mittels einer methanolischen Calciumchloridlösung beeinflußt (J. Am. Chem. Soc. 73, 4212/1951/ ; US-Patentschriften Nr. 2.658.078 und 2.915.555). Prinzipiell lassen sich Hydrochloride auch durch Umsetzen der Ausgangsverbindung mit einem anderen Hydrochlorid erhalten, jedoch haben diese Verfahren in die Praxis keinen Eingang gefunden und spielen nur eine untergeordnete Rolle, weil von allen Salzen die Hydrochloride die löslichsten sind und ihre Isolierung durch einfaches Ausfällen oder Kristallisieren daher nicht realisierbar ist.

Die Bildung von Hydrochloriden kann auch in nichtprotischen Lösungsmitteln oder ohne Lösungsmittel eintreten ; in diesem Falle wird das vom HCl-Molekül abdissoziierende Proton von dem einsamen Elektronenpaar des Stickstoffatoms der Anhydrobase gebunden (The Chemistry of the Amino Group, Ed. by S. Patai, Interscience, London 1968).

Es sind zahlreiche Hydrochloride bekannt, die mit Kristallwasser kristallisieren. Das Oxytetracyclinhydrochrorid kristallisiert zum Beispiel — wenn genügend Wasser vorhanden ist — mit 3 Molekülen Kristallwasser (ungarische Patentschrift Nr. 143.911). Diese Hydrochloride sind in organischen Lösungsmitteln unter wasserfreien Bedingungen wesentlich besser löslich als in Gegenwart von Wasser. Diese Eigenschaft kann zur Reinherstellung der Hydrochloride genutzt werden. Gemäß der belgischen Patentschrift Nr. 638.881 wird die Ausgangssubstanz des Oxytetracyclinhydrochlorids (ein Oxytetracyclin-Komplex) unter wasserfreien Bedingungen mit salzsäurem Methanol behandelt, die in der Hydrochloridlösung enthaltenen festen Verunreinigungen werden abfiltriert, und dann wird das reine Oxytetracyclinhydrochlorid durch Zusatz konzentrierter wäßriger Salzsäure ausgefällt.

Wird die Salzbildung in wäßrigem Medium vorgenommen, so muß auf jeden Fall eingedampft oder aufkonzentriert werden. Dabei kann es im Falle empfindlicher organischer Verbindungen zu Zersetzungsreaktionen kommen, deren Produkte das Hydrochlorid verunreinigen. Das Lyophilisieren der Lösungen erfordert teure Vorrichtungen und ist außerdem energieaufwendig. Durch Einleiten von trockenem Salzsäuregas in wasserfreie Lösungen beziehungsweise durch Einsatz HCl-haltiger Lösungsmittel (zum Beispiel ethanolischer Salzsäure) können zwar gute Ausbeuten erzielt werden, jedoch kann die Herstellung und Handhabung trockenen Salzsäuregases nicht als eine für die Industrie annehmbare Lösung betrachtet werden. Die Umsetzung mit einem anderen Hydrochlorid wird im allgemeinen in wässrigem Medium ausgeführt und hat daher die oben bereits erwähnten Nachteile.

Aufgabe der Erfindung ist die Bereitstellung eines einfachen und wirtschaftlichen Verfahrens zur Herstellung von Hydrochloriden von ein protonierbares Stickstoffatom enthaltenden Verbindungen in hoher Ausbeute und Reinheit.

Die Erfindung beruht auf der Erkenntnis, daß aus Verbindungen der allgemeinen Formel

$$Q{-}SO_2{-}Cl$$

worin Q für Hydroxylgruppe, Alkylgruppe mit 1-4 Kohlenstoffatomen, für Arylgruppe oder durch $C_{1-4}$-Alkylgruppe substituierte Arylgruppe steht, durch Zusatz eines Alkohols Chlorwasserstoff freigesetzt wird und diese Reaktion in einem geeignet gewählten Lösungsmittelmedium zur Bildung von Hydrochloriden geeignet ist.

Gegenstand der Erfindung ist demnach ein Verfahren zur Herstellung von Hydrochloriden von polaren, wasserlöslichen Verbindungen mit einem protonierbaren Stickstoffatom in einem Lösungsmittel, das dadurch gekennzeichnet ist, daß man folgende Stufen durchführt :

a) Behandeln der polaren, wasserlöslichen Verbindung mit einem protonierbaren Stickstoffatom oder einem Salz davon mit dem Chlorwasserstoff enthaltenden Reaktionsprodukt einer Sulfonylverbindung der Formel (I) $Q—SO_2—Cl$, worin Q eine Hydroxylgruppe, eine $C_1$-$C_4$-Alkylgruppe, eine Arylgruppe oder eine $C_1$-$C_4$-Alkylarylgruppe bedeutet, und einem Alkohol, ausgewählt aus der Gruppe aliphatische Alkohole, cycloaliphatische Alkohole, aliphatische Diole oder Triole, aromatische Alkohole oder Mischungen davon, in einem Lösungsmittel oder Lösungsmittelgemisch, in welchem das Reaktionsprodukt hergestellt wurde durch Auflösen der Verbindung der Formel (I) in einem Überschuß des Alkohols in einer Menge von 0,1 bis 10 Mol/l, und worin die Löslichkeit des so hergestellten Hydrochloridsalzes wenigstens 0,1 Gew.% in dem Lösungsmittel oder Lösungsmittelgemisch beträgt, und

b) Gewinnen des Hydrochloridsalzes.

Aus der Sulfonylverbindung $Q—SO_2—Cl$ und dem Alkohol bilden sich intermediär Chlorwasserstoff und ein Schwefelmonoester. Der im Überschuß eingesetzte Alkohol hält beide Reaktionsprodukte in Lösung. Verwendet man als Sulfonylverbindung Chlorsulfonsäure (Q = OH) und als Alkohol ein Alkanol, so bildet sich ein Schwefelsäure-monoalkylester. Diese noch ein saures Proton enthaltende Verbindung ist als Säure eine Größenordnung schwächer als Salzsäure. Die bei der Verwendung von Methansulfonylchlorid (Q = $CH_3$) oder p-Toluol-sulfonylchlorid (Q = p-Toluyl) und einem Alkanol entstehenden Methansulfonsäure- beziehungsweise p-Toluolsulfonsäure-alkylester haben keinen Säurecharakter. Durch entsprechende Auswahl des Alkohols und der Sulfonylverbindung sowie des Lösungsmittels kann in jedem Falle erreicht werden, daß das Hydrochlorid ausfällt, während der gebildete Monoester in Lösung bleibt.

Als Verbindungen der Formel $Q—SO_2—Cl$ werden bevorzugt die oben bereits genannten verwendet.

Als Alkoholkomponente verwendet man aliphatische Alkohole wie Methanol, Ethanol oder Propanol, aliphatische Diole oder Triole, zum Beispiel Ethylenglykol, cycloaliphatische Alkohole, zum Beispiel Cyclohexanol, oder aromatische Alkohole wie Benzylalkohol, oder Gemische der aufgeführten Alkohole.

Als zur Bildung von Hydrochloriden geeignete Verbindungen seien beispielsweise die folgenden genannt : Amine, zum Beispiel aliphatische, aromatische Amine, Cycloalkyl-, Aralkyl-, Heterocycloalkylamine, heteroaromatische Amine, Diazoamin (Triazen) und ihre Derivate ; Hydroxylamin und seine Derivate ; Hydrazin und seine Derivate ; Carbonsäureimidat, Carbonsäureamidin, Carbazide, Semicarbazide, Guanidin, Aminosäuren, Aminozucker und ihre Derivate ; ein oder mehrere Heteroatome enthaltende, aromatische oder teilweise oder völlig gesättigte Ringe mit 5 Gliedern, zum Beispiel Pyrrol, Oxazol, Isoxazol, Thiazol, Isothiazol, Imidazol, Pyrazol, Oxadiazol, Thiadiazol, Triazol, Tetrazol und ihre Derivate ; aromatische oder teilweise oder vollständig gesättigte, ein oder mehrere Stickstoffatome und gegebenenfalls nach andere Heteroatome enthaltende Ringe mit 6 Gliedern, zum Beispiel Pyridin, Pyridazin, Pyrimidin, Pyrazin, Oxadiazin, Thiadiazin, Triazin, Tetrazin und ihre Derivate ; ein oder mehrere Stickstoffatome und gegebenenfalls noch weitere Heteroatome enthaltende, gesättigte oder ungesättigte cyclische Verbindungen mit 6-10 gliedern, zum Beispiel Azepin, Oxazepin, Thiazepin, Diazepin, Oxadiazepin, Thiadiazepin, Azocin, Azonin, Azecin, Acridin und ihre Derivate ; Alkaloide, Steroide, basische oder amphotere Farbstoffe und ihre Derivate ; basische oder amphotere Antibiotica (zum Beispiel Klindamycin, Streptomycin, Neomycin, Tobramycin), einen β-Lactamring enthaltende Antibiotica (Pivampicillin, Cephalexin), Tetracycline (Oxytetracyclin und seine Komplexe, Chlortetracyclin, Tetracylcin, Doxicyclin), Anthramycine (Daunomycin, Adriamycin, Aklavinon), Cyclopeptide (Enduracidin) Macrolide (Erythromycin, Nistatin, Oleandomycin) und ihre Derivate.

Das erfindungsgemäße Verfahren wird in einem Lösungsmittelmedium durchgeführt. Als Lösungsmittel kann ein Überschuß des eingesetzten Alkohols dienen. Geeignete Lösungsmittelmedien sind auch weitere Alkohole, Ester, Ketone oder Ether sowie die gegebenenfalls auch Wasser enthaltenden Gemische dieser Lösungsmittel.

Das herzustellende Hydrochlorid maß in dem verwendeten Lösungsmittelmedium zu wenigstens 0,1 Gew.-% löslich sein. Die Verbindung $Q—SO_2—Cl$ wird in dem als Reaktionskomponente dienenden Alkohol gelöst. Die Konzentration dieser Lösung beträgt 0,1-10 Mol/Liter, vorzugsweise 0,5-2 Mol/Liter.

Mit dem erfindungsgemäßen Verfahren können die Hydrochloride mit guter Ausbeute in hochreiner Form auf einfache, auch industriell leicht durchführbare Weise gewonnen werden.

Das erfindungsgemäße Verfahren wird an Hand der folgenden Beispiele näher erläutert, ist jedoch nicht auf diese Beispiele beschränkt.

## Beispiel 1

150 ml Methanol und 50 g Oxytetracylin-Calciumsilikat-Komplex (enthaltend 25 g Oxytetracyclin) werden bei Raumtemperatur 30 Minuten lang gerührt. Zu dem Gemisch wird innerhalb von 30 Minuten die Lösung von 15 g wasserfreiem Calciumchlorid in 100 ml Methanol gegeben. Das Gemisch wird eine weitere halbe Stunde lang gerührt. Dann wird der pH-Wert des Gemisches durch langsame Zugabe einer methanolischen Chlorsulfonsäurelösung der Konzentration 1,5 Mol/Liter auf 3,5 eingestellt, wozu etwa 34 ml erforderlich sind (Kontrolle mit pH-Meßgerät und Glaselektrode). Nach Zugabe von 1 g Aktivkohle wird das Gemisch 30 Minuten lang gerührt, dann filtriert und der Filterkuchen mit der Lösung von 3,75 g wasserfreiem Calciumchlorid in 50 ml Methanol gewaschen. Die Waschflüssigkeit wird mit dem Filtrat

vereinigt. Unter Rühren und Kühlen werden zu der Lösung innerhalb von 15 Minuten 17 ml 37 %ige Salzsäure gegeben, wodurch sich ein pH-Wert von 0,4 einstellt. Inzwischen gibt man bei pH 1,5 0,2 g reines Oxytetracyclin-hydrochlorid als Impfkristalle zu. Sofort beginnt die Kristallausscheidung. Das Gemisch wird noch 30 Minuten lang gerührt und dann filtriert. Die nadelförmigen Kristalle werden in einem Gemisch aus 25 ml kaltem Methanol und 2,5 ml 37 %iger Salzsäure suspendiert, nach dem Dekantieren noch zweimal mit je 10 ml des gleichen Gemisches gewaschen, dann in 25 ml kaltem Methanol suspendiert und anschließend noch 2x mit je 10 ml kaltem Methanol gewaschen. Das Produkt wird bei 50 °C an der Luft getrocknet. Man erhält 23,4 g Oxytetracyclin-hydrochlorid in Form gelber, nadelförmiger Kristalle, die 90 % Oxytetracyclin und 7 % Wasser enthalten. Das entspricht, auf den eingesetzten Komplex bezogen, einer Ausbeute von 78 %.

Beispiel 2

Zu der Lösung von 20 g trockenem, in Nadelkristallform vorliegendem Oxytetracyclin-hydrochlorid (93 %ig) in 37 ml 0,1 M Salzsäure und 56 ml Methanol wird unter Rühren 1 g Aktivkohle gegeben. Die Lösung wird einige Minuten lang gerührt und dann filtriert. Zu dem Filtrat werden unter Rühren und Kühlen bei 5-10 °C 108 ml methanolische Chlorsulfonsäurelösung der Konzentration 1,63 Mol/Liter gegeben. Das kristallisierende Gemisch wird bei der angegebenen Temperatur noch 30 Minuten lang gerührt und dann filtriert. Das Produkt wird in 20 ml verdünnter methanolischer Salzsäure suspendiert, filtriert und mit 10 ml verdünnter methanolischer Salzsäure erneut gewaschen. Nach dem Filtrieren wird einmal mit 20 ml, danach mit 10 ml kaltem Methanol gewaschen. Schließlich wird das Produkt bei 50 °C an der Luft getrocknet. Man erhält 16,76 g gelbes, kristallines Oxytetracyclin-hydrochlorid (Ausbeute : 82 %), das 91 % Substanz und 8 % Wasser enthält.

Beispiel 3

20 g rohes Oxytetracyclin-dihydrat (85 %ig) werden auf die im Beispiel 2 beschriebene Weise behandelt. Man erhält 15,13 g gelbes, kristallines Oxytetracyclin-hydrochlorid (Ausbeute : 81 %), das 91 % Oxytetracyclin-hydrochlorid und 8 % Wasser enthält.

Beispiel 4

Die mit 8 ml Wasser bereitete Lösung von 10 g Betain (Carboxymethyl-trimethyl-ammoniumchlorid) wird filtriert und dann bei Raumtemperatur mit 60 ml äthanolischer Chlorsulfonsäurelösung der Konzentration von 1,5 Mol/Liter versetzt. Die kristallisierende Lösung wird auf 0 °C gekühlt und bei dieser Temperatur eine Stunde lang gerührt. Das Produkt wird abfiltriert, mit n-Propanol gewaschen und dann getrocknet. Man erhält 11,2 g (86 %) Betain-hydrochlorid in Form weißer Kristalle, die bei 231-232 °C schmelzen.

Beispiel 5

Zu der mit 50 ml Diethylether bereiteten Lösung von 9,8 ml (10,8 g) Phenylhydrazin werden unter Rühren 27 ml einer äthanolischen Methansulfonylchloridlösung der Konzentration von 4 Mol/Liter gegeben. Das kristallisierende Gemisch wird bei Raumtemperatur eine halbe Stunde lang gerührt. Das kristalline Produkt wird filtriert, mit Diethyläther gewaschen und dann getrocknet. 13,6 g (94 %) Phenylhydraziniumchlorid werden erhalten. Das Produkt schmilzt bei 245-246 °C.

Beispiel 6

Die Lösung von 9,7 g Tobramycin in 40 ml 80 %igem Methanol wird filtriert und dann unter Rühren bei Raumtemperatur mit 40 ml einer methanolischen Methansulfonylchloridlösung der Konzentration 0,5 Mol/Liter versetzt. Ein weißer Niederschlag fällt aus. Das Gemisch wird 15 Minuten lang gerührt. Dann wird das Produkt abfiltriert, mit Methanol gewaschen und im Vakuum bei 60 °C getrocknet.

13,1 g Tobramycin-hydrochlorid (98 %) werden erhalten. $R_f$ = 0,34 und stimmt mit dem $R_f$-Wert einer Referenzprobe überein (Dünnschichtchromatographie an Fertigplatten 20 × 20 cm, Merck Kieselgel 60 $F_{254}$ ; Laufmittel : mit 30 %igem Ethanol bereitete NaCl-Lösung der Konzentration 2,5 M, Entwickler : 0,5 %iger wässrige Natriumhypochloritlösung, Trocknen, Besprühen mit Ethanol, Trocknen, Besprühen mit einer 1,1 % Cadmiumjodid und 1,5 % Stärke (Amylose) enthaltenden Lösung).

Beispiel 7

Zu 15,3 g Dopamin (3,4-Dihydroxyphenäthylamin) werden unter Stickstoffatmosphäre 50 ml Diethylether gegeben und dann unter Rühren 55 ml methanolische p-Toluolsulfonyl-chloridlösung der Konzentration 2 Mol/Liter dosiert. Das Gemisch wird bei Raumtemperatur eine Stunde lang gerührt. Die ausgeschiedenen Kristalle werden abfiltriert, mit Ether gewaschen und dann getrocknet. Man erhält 17,3

g (91 %) Dopamin-hydrochlorid, das bei 240-241°C schmilzt.

Beispiel 8

Zu einem Gemisch von 15,2 g Morphin und 30 ml 80 %igem Ethanol werden unter Rühren 30 ml einer Ethanolischen p-Toluolsulfonylchloridlösung der Konzentration von 2 Mol/Liter gegeben. Das Gemisch wird bei Raumtemperatur 2 Stunden lang, dann bei 5° C noch 30 Minuten lang gerührt. Das kristalline Produkt wird abfiltriert, mit 2-Propanol gewaschen und dann getrocknet. Man erhält 18,2 g (97 %) Morphinhydrochlorid, das bei 198-200 °C schmilzt. $[\alpha]_D^{25}$ : —113,5° (c = 2,2 % bezogen auf die wasserfreie Base ; Wasser).

Beispiele 9-31

Auf die in den Beispielen 4-8 beschriebene Weise werden die in der folgenden Tabelle aufgeführten Verbindungen hergestellt. Die eingesetzten Reagenzien, Lösungsmittel, Reaktionsbedingungen, Ausbeuten und Schmelzpunkte sind ebenfalls in der Tabelle angegeben, wobei die folgenden Abkürzungen und Symbole verwendet wurden :

Nr. Nummer des Beispiels

A Bezeichnung des Produktes
B Verbindungstyp der Ausgangsverbindung
C Reaktionsmedium der Salzbildung
D Angabe des Reagens $Q—SO_2—Cl$
E Alkoholkomponente für die Reaktion mit $Q—SO_2—Cl$
F Ausbeute an Hydrochlorid, %
G Schmelzpunkt des Hydrochlorids, °C
H Spezifisches Drehvermögen des Hydrochlorids, $[\alpha]_D^{25}$

MeCH = Methanol
1-PrOH = n-Propanol
1-BuOH = n-Butanol
$Me_2CO$ = Aceton
EtOH = Ethanol
2-PrOH = Isopropanol
AcOEt = Ethylacetat
$Et_2O$ = Diethylether
CS = Chlorsulfonsäure
MsCl = Methansulfonylchlorid
TsCl = p-Toluolsulfonylchlorid

| Nr. | A | B | C | D | E | F | G | H |
|---|---|---|---|---|---|---|---|---|
| 9 | Propranolol .HCl | Aliphatitisches Amin | 1-PrOH | CS | 1-PrOH | 93 | 163-4 | — |
| 10 | Chlorguanid .HCl | Guanidinderivat | EtOH 90 % | CS | EtOH | 96 | 243-4 | |
| 11 | Penicillamin .HCl | Aminosäure | AcOEt | CS | 2-PrOH | 91 | 177-8 | −63° c = 5, NaOH |
| 12 | Procain.HCl | Aromatisches Amin | 1-PrOH | CS | EtOH | 88 | 153-6 | |
| 13 | Ketamin.HCl | Cycloalkylamin | EtOH | CS | EtOH | 85 | 263-3 | |
| 14 | Amantadin.HCl | Cycloalkylamin | EtOH + $Et_2O$ | CS | EtOH | 94 | 360 | |
| 15 | Klindamycin .HCl.$H_2O$ | Antibiotikum Aminocyclitol | EtOH + AcOEt | MsCl | EtOH | 94 | 141-3 | 144° Wasser |
| 16 | Streptomycin .3HCl | Antibiotikum Aminocyclitol | EtOH | MsCl | EtOH | 88 | | −84° |
| 17 | Pivampicillin. HCl | Antibiotikum ß-Lactam | 1-PrOH | CS | 1-PrOH | 85 | 154-6 | 200° c = 1, $H_2O$ |
| 18 | Noformycin .2HCl | Antibiotikum Aminosäure | MeOH | CS | MeOH | 92 | 262-4 | 8,8° MeOH |

(Fortsetzung)

| Nr. | A | B | C | D | E | F | G | H |
|---|---|---|---|---|---|---|---|---|
| 19 | Spectinomycin .2HCl | Antibiotikum | EtOH + Wasser | FsCl | EtOH | 91 | — | – 20° |
| 20 | Doxicyclin .HCl | Antibiotikum Tetracyclin | EtOH | CS | EtOH | 92 | 199-202 | – 110° |
| 21 | Tetracyclin .HCl | Antibiotikum Tetracyclin | 1-BuOH | FsCl | 1-BuOH | 96 | 213-4 | – 257,5° |
| 22 | Doxorubicin .HCl | Antibiotikum Anthracyclin | $Me_2CO$ | CS | MeOH | 92 | 204-5 | 243°c 0,1 % MeOH |
| 23 | Cleandomy-mycin.HCl | Antibiotikum Macrolid | AcOEt | MsCl | EtOH | 90 | 133-5 | – 54° MeOH |
| 24 | Tonzilamin .HCl | Heteroarylamin | EtOH + $Et_2O$ | MsCl | EtOH | 95 | 174-6 | |
| 25 | Metidylazin .HCl | Pyrrol-derivat | 2-PrOH | FsCl | 2-PrOH | 93 | 187-9 | |
| 26 | Tetramizol .HCl | Imidazol-derivat | EtOH | TsCl | EtOH | 84 | 264-6 | |
| 27 | Phenglutarimid .HCl | Pyridin-derivat | MeOH + AcOEt | CS | MeOH | 91 | 176-7 | |
| 28 | Opipramol .HCl | Pyrazin- und Azepinderiv. | EtOH | CS | EtOH | 94 | 227-9 | |
| 29 | Nalorfin .HCl | Alkaloid | EtOH | CS | EtOH | 94 | 270 | |
| 30 | Hydrocortamat .HCl | Steroid | AcOEt | CS | EtOH | 92 | 222 | |
| 31 | Cyproheptadin .HCl | Pyridin-derivat | EtOH + | CS | EtOH | 88 | 252-4 | |

**Patentansprüche**

1. Verfahren zur Herstellung von Hydrochloriden von polaren, wasserlöslichen Verbindungen mit einem protonierbaren Stickstoffatom in einem Lösungsmittel, dadurch gekennzeichnet, daß man folgende Stufen durchführt :

a) Behandeln der polaren, wasserlöslichen Verbindung mit einem protonierbaren Stickstoffatom oder einem Salz davon mit dem Chlorwasserstoff enthaltenden Reaktions-produckt einer Sulfonylverbindung der Formel (I) Q—$SO_2$—Cl worin Q eine Hydroxylgruppe, eine $C_1$-$C_4$-Alkylgruppe, eine Arylgruppe oder eine $C_1$-$C_4$-Alkylarylgruppe bedeutet, und einem Alkohol, ausgewählt aus der Gruppe aliphatische Alkohole, cycloaliphatische Alkohole, aliphatische Diole oder Triole, aromatische Alkohole oder Mischungen davon, in einem Lösungsmittel oder Lösungsmittelgemisch, in welchem das Reaktionsprodukt hergestellt wurde durch Auflösen der Verbindung der Formel (I) in einem Überschuß des Alkohols in einer Menge von 0,1 bis 10 Mol/l, und worin die Löslichkeit des so hergestellten Hydrochloridsalzes wenigstens 0,1 Gew.% in dem Lösungsmittel oder Lösungsmittelgemisch beträgt, und

b) Gewinnen des Hydrochloridsalzes.

2. Verfahren nach Anspruch 1, dadurch gekennzeichnet, daß man als polare wasserlösliche Verbindungen mit einem protonisierbaren Stickstoffatom Amine Hydroxyamine, Hydrazin, Aminosäuren, Aminozucker, heterocyclische Verbindunge mit 5 bis 12 Gliedern im Ringsystem, Antibiotika oder die Derivate der aufgeführten Verbindungsklassen verwendet.

3. Verfahren nach Anspruch 2, dadurch gekennzeichnet, daß man Antibiotika der Gruppen Aminocyclitole, β-Lactamantibiotika, Tetracycline, Anthracyline, Cyclopeptide oder Macrolide verwendet.

4. Verfahren nach einem der Ansprüche 1 bis 3, dadurch gekennzeichnet, daß man als Lösungsmittel Gemische des verwendeten Alkohols mit Ethern, Ketonen, Estern, sonstigen Alkoholen und/oder Wasser verwendet.

5. Verfahren nach einem der Ansprüche 1 bis 4, dadurch gekennzeichnet, daß man als Sulfonylverbindung der allegemeinen Formel Q—$SO_2$—Cl Chlorsulfonsäure, Methansulfonylchlorid oder p-Toluolsulfonylchlorid verwendet.

6. Verfahren nach einem der Ansprüche 1 bis 5, dadurch gekennzeichnet, daß man einen aliphatischen Alkohol mit 1 bis 4 Kohlenstoffatomen verwendet.

7. Verfahren nach einem der Ansprüche 1 bis 6, dadurch gekennzeichnet, daß man aus der Verbindung Q—$SO_2$—Cl und dem Alkohol eine Lösung mit einer Konzentration von 0,5 bis 2 Mol/Liter bereitet und diese mit der zur Bildung eines Hydrochlorides geeigneten Verbindung umsetzt.

**Claims**

1. Process for the preparation of hydrochlorides of polar, water-soluble compounds having a protonatable nitrogen atom in a solvent, characterised in that the following steps are carried out :

a) treatment of the polar, water-soluble compound having a protonatable nitrogen atom, or a salt thereof, with a reaction product, containing hydrogen chloride, of a sulphonyl compound of the formula (I) Q—$SO_2$—Cl, in which Q represents a hydroxy group, a $C_1$-$C_4$-alkyl group, an aryl group or a $C_1$-$C_4$-alkylaryl group, and an alcohol selected from the group comprising aliphatic alcohols, cycloaliphatic alcohols, aliphatic diols or triols, aromatic alcohols or mixtures thereof, in a solvent or solvent mixture in which the reaction product has been prepared by dissolving the compound of the formula (I) in an excess of the alcohol in an amount of from 0.1 to 10 mol/l, and in which the solubility of the hydrochloride salt so prepared is at least 0.1% by weight in the solvent or solvent mixture, and

b) recovering the hydrochloride salt.

2. Process according to claim 1, characterised in that amines, hydroxyamines, hydrazine, amino acids, amino sugars, heterocyclic compounds having from 5 to 12 members in the ring system, antibiotics or derivatives of the mentioned compound classes are used as the polar warter-soluble compounds having a protonatable nitrogen atom.

3. Process according to claim 2, characterised in that antibiotics from the groups aminocyclitols, β-lactam antibiotics, tetracyclines, anthracyclines, cyclopeptides or macrolides are used.

4. Process according to any one of claims 1 to 3, characterised in that mixtures of the alcohol used with ethers, ketones, esters, other alcohols and/or water are used as solvent.

5. Process according to any one of claims 1 to 4, characterised in that chlorosulphonic acid, methanesulphonyl chloride or p-toluenesulphonyl chloride is used as the sulphonyl compound of the general formula Q—$SO_2$—Cl.

6. Process according to any one of claims 1 to 5, characterised in that an aliphatic alcohol having from 1 to 4 carbon atoms is used.

7. Process according to any one of claims 1 to 6, characterised in that a solution having a concentration of from 0.5 to 2 mol/litre is prepared from the compound Q—$SO_2$—Cl and the alcohol, and this solution is reacted with a compound suitable for the formation of a hydrochloride.

**Revendications**

1. Procédé de préparation de chlorhydrates de composés polaires hydrosolubles ayant un atome d'azote protonable, dans un solvant, caractérisé en ce que l'on met en œuvre les stades suivants :

a) le traitement du composé polaire hydrosoluble ayant un atome d'azote protonable ou d'un sel dudit composé, avec le produit réactionnel, contenant de l'acide chlorhydrique, d'un composé sulfonylé de formule (I) Q—$SO_2$—Cl, dans laquelle Q représente un groupe hydroxyle, un groupe alcoyle en $C_1$-$C_4$, un groupe aryle ou un groupe alcoyl(en $C_1$-$C_4$)-aryle, et d'un alcool choisi dans le groupe des alcools aliphatiques, des alcools cycloaliphatiques, des diols ou triols aliphatiques, des alcools aromatiques ou des mélanges de ceux-ci, dans un solvant ou un mélange de solvants dans lequel le produit réactionnel a été préparé par dissolution du composé de formule (I) dans un excès de l'alcool en une quantité de 0,1 à 10 moles/l, la solubilité du chlorhydrate salin ainsi préparé s'élevant au moins à 0,1 % en poids dans le solvant ou le mélange de solvants, et

b) l'isolement du chlorhydrate salin.

2. Procédé selon la Revendication 1, caractérisé en ce qu'on utilise comme composés polaires hydrosolubles ayant un atome d'azote protonable, des amines, des hydroxyamines, l'hydrazine, des aminoacides, des aminosucres, des composés hétérocycliques contenant 5 à 12 chaînons dans le système cyclique, des antibiotiques ou les dérivés des classes de composés citées.

3. Procédé selon la Revendication 2, caractérisé en ce que l'on utilise des antibiotiques des groupes aminocyclitols, antibiotiques à noyau β-lactame, tétracyclines, anthracyclines, cyclopeptides ou macrolides.

4. Procédé selon l'une des Revendications 1 à 3, caractérisé en ce qu'on utilise comme solvant des mélanges de l'alcool utilisé avec des éthers, des cétones, des esters, d'autres alcools et/ou de l'eau.

5. Procédé selon l'une des Revendications 1 à 4, caractérisé en qu'on utilise comme composé sulfonylé de formule générale Q—$SO_2$—Cl, l'acide chlorosulfonique, le chlorure de méthanesulfonyle ou le chlorure de p-toluènesulfonyle.

6. Procédé selon l'une des Revendications 1 à 5, caractérisé en qu'on utilise un alcool aliphatique ayant 1 à 4 atomes de carbone.

7. Procédé selon l'une des Revendications 1 à 6, caractérisé en ce qu'en partant du composé Q—$SO_2$—Cl et de l'alcool, on prépare une solution d'une concentration de 0,5 à 2 moles/litre et que l'on fait réagir celle-ci avec le composé propre à la formation d'un chlorhydrate.